(19) **Europäisches Patentamt European Patent Office Office européen des brevets**

(11) **EP 4 422 565 B1**

(12) **EUROPEAN PATENT SPECIFICATION**

(45) Date of publication and mention
of the grant of the patent:
**22.04.2026   Bulletin 2026/17**

(21) Application number: **22813151.2**

(22) Date of filing: **28.10.2022**

(51) International Patent Classification (IPC):
**A61F 5/01** (2006.01)

(52) Cooperative Patent Classification (CPC):
**A61F 5/013;** A61F 2005/0155; A61F 2005/0167

(86) International application number:
**PCT/EP2022/080205**

(87) International publication number:
**WO 2023/073173 (04.05.2023 Gazette 2023/18)**

(54) **ELBOW ORTHOSIS AND PROCESS FOR ADAPTING A CONFIGURATION OF SUCH AN ORTHOSIS TO A PATIENT**

ELLBOGENORTHESE UND VERFAHREN ZUR ANPASSUNG EINER KONFIGURATION SOLCH EINER ORTHESE AN EINEN PATIENTEN

ORTHÈSE DE COUDE ET PROCÉDÉ D'ADAPTATION D'UNE CONFIGURATION D'UNE TELLE ORTHÈSE POUR UN PATIENT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **29.10.2021 EP 21306527**

(43) Date of publication of application:
**04.09.2024   Bulletin 2024/36**

(73) Proprietors:
• **INSTITUT NATIONAL DE LA SANTE ET DE LA RECHERCHE MEDICALE (INSERM)**
  **75013 Paris (FR)**
• **ASSISTANCE PUBLIQUE-HOPITAUX DE PARIS**
  **75012 Paris 12 (FR)**
• **Université Versailles Saint-Quentin-en-Yvelines**
  **78000 Versailles (FR)**
• **Lexat**
  **78440 Gergenville (FR)**

(72) Inventors:
• **CARTA, Jean-Paul**
  **78440 Gargenville (FR)**

• **CHARLES, Sébastien**
  **78140 Velizy Villacoublay (FR)**
• **PRADEL, Gilbert**
  **92340 Bourg-La-Reine (FR)**
• **BARBOT, Frédéric**
  **92380 Garches (FR)**

(74) Representative: **Casalonga**
  **Bayerstraße 71/73**
  **80335 München (DE)**

(56) References cited:
**EP-A1- 3 143 970    KR-A- 20200 067 443**
**US-B1- 6 203 511    US-B1- 6 993 808**

• **RIPEL TOMAS ET AL: "Active Elbow Orthosis", INTERNATIONAL JOURNAL OF ADVANCED ROBOTIC SYSTEMS, vol. 11, no. 9, 8 September 2014 (2014-09-08), CR, pages 1 - 10, XP055910323, ISSN: 1729-8814, DOI: 10.5772/58874**

**Description**

The present invention concerns an elbow orthosis, which can be used for the rehabilitation of the arm of patients affected by spasticity, for instance after a cardio-vascular accident or CVA.

One issue with these patients is that they cannot fully control their muscles, so that re-education must start with repeated movements of flexion/extension of the elbow.

In this context, several elbow-orthoses are known for moving the forearm of a patient with respect to his/her arm, thanks to two fittings articulated together and respectively mounted around the arm and around the forearm of the patient.

For instance, the Active Elbow Orthosis mentioned in the article of Riple et al published in the International Journal of Advanced Robotic Systems (2014, 11:143) includes two fittings respectively configured to be mounted on an arm and on a forearm of a patient, an electric actuator and a worm-and-wheel transmission for transmitting the output movement of the actuator to a fitting attached to the patient's forearm. This orthosis includes a safety mechanical stop arrangement for limiting the angular relative movement between the two fittings. This safety mechanical stop arrangement includes two aluminum switches whose position must be set in advance, which is cumbersome and not intuitive. In addition, this mechanism is complex, thus relatively expensive. The adaptation of the configuration of the orthosis to its actual use, in particular for a left or right arm, takes time and requires a highly qualified operator.

US6203511B1 discloses an orthotic joint, suable for a knee, ahip or an ankle. US6993808 discloses adjustable hinges for orthopedic knee and elbow splints. EP314970 discloses a motorized articulated joint. KR20200067446 discloses a medical joint, usable for an elbow. All these mechanical devices are difficult to set to their respective conditions of use.

The present invention aims at solving these problems by providing a new elbow orthosis with an improved mechanical stop arrangement, which facilitates the setting of the orthosis for the angular relative movement between the first and second fittings of the orthosis.

To this end, the present invention relates to an elbow orthosis comprising a first fitting configured to be mounted on an arm of a patient, a second fitting configured for being mounted on a forearm of the patient, an articulation mechanism between the first and second fittings and an electric gear-motor mounted on the first fitting for moving the second fitting with respect to the first fitting in rotation around a first rotation axis of the articulation mechanism. An output shaft of the electric gear-motor is aligned on a second rotation axis perpendicular to the first rotation axis. The orthosis is equipped with an angular sensor for sensing the angular position of the second fitting with respect to the first fitting around the first rotation axis, with a torque sensor for sensing a torque exerted on the articulation mechanism by the electric gear-motor and with a rotation shaft. A worm-and-wheel transmission drives the rotation shaft, which defines the first rotation axis, from the rotational movement of the output shaft of the electric gear-motor. The second fitting is secured to the rotation shaft. A mechanical stop arrangement is configured for limiting the angular movement of the second fitting with respect to the first fitting around the first rotation axis. The mechanical stop arrangement includes a first plate, with a first circular slot, and a crank secured to the rotation shaft and equipped with a pin engaged in the first circular slot. According to the invention,

- the first plate is provided with a first series of through holes spread on a circle centered on a central axis of the first circular slot;
- the mechanical stop arrangement includes a second plate provided with a second circular slot and a second series of through holes spread on a circle centered on a central axis of the second circular slot; and
- the pin of the crank is engaged in the second circular slots;
- the central axis of the first circular slot and the central axis of the second circular slot of the first and second plates are aligned on the first rotation axis and the relative orientation of the first and second plates around this first rotation axis is set by aligning at least one through hole of the first series with one through hole of the second series;
- some portions of the first and second circular slots overlap, depending on the orientation of the first and second plates around the common axis; and
- the respective extremities of the overlapping portions of the first and second circular slots define the amplitude of the maximum angular movement of the second fitting with respect to the first fitting, around the first rotation axis, when it is driven by the worm-and-wheel transmission.

Owing to the invention, the elbow orthosis may be easily adapted to the morphology and pathology of a patient, in particular for what concerns the end positions of the angular movements between the first and second fittings and the choice between his/her left and right arms.

According to advantageous and non-compulsory aspects of the invention, the elbow orthosis of the invention might incorporate the features of any one of claims 2 to 11.

According to another aspect, the present invention relates to a process for adapting a configuration of an elbow orthosis of the type mentioned here-above to the morphology and/or pathology of a patient, this method including at least the following steps:

a) adjusting the respective angular position of the first and second plates around their common central axis, so as to define with their overlapping first and second circular slots a path for the pin mounted on the crank secured to the rotation shaft; and

b) immobilizing the first and second plates in their respective angular positions by insertion of a locking member in one through hole of the first series and in one through hole of the second series.

According to advantageous and non-compulsory aspects of the invention, this process might incorporate the features of any one of claims 13 to 15.

The invention will be better understood on the basis of the following description of an elbow orthosis according to the invention, given as an illustrative example only, in reference to the annexed drawings where:

- Figure 1 is a side view of an orthosis according to the invention configured for use with a right arm of a patient and represented in a flexed position;
- Figure 2 is a side view similar to figure 1 when the orthosis is in an extended position;
- Figure 3 is a side view comparable to figure 1 when the orthosis is configured for use on the left arm of a patient and represented in a flexed position;
- Figure 4 is a side view similar to figure 3 when the orthosis is in an extended position;
- Figure 5 is a perspective view of the orthosis in the configuration and position of figure 3, insert A corresponding to a partial cut view in the direction of arrows A-A on figure 5;
- Figure 6 is an exploded perspective view of the orthosis of figures 1 to 5, seen from an angle different from the one of figure 5;
- Figure 7 is an enlarged perspective view of detail VII on figure 6 and
- Figure 8 is a representation of the use of the orthosis of figures 1 to 7 on the right arm of a patient laying on a medical bed.

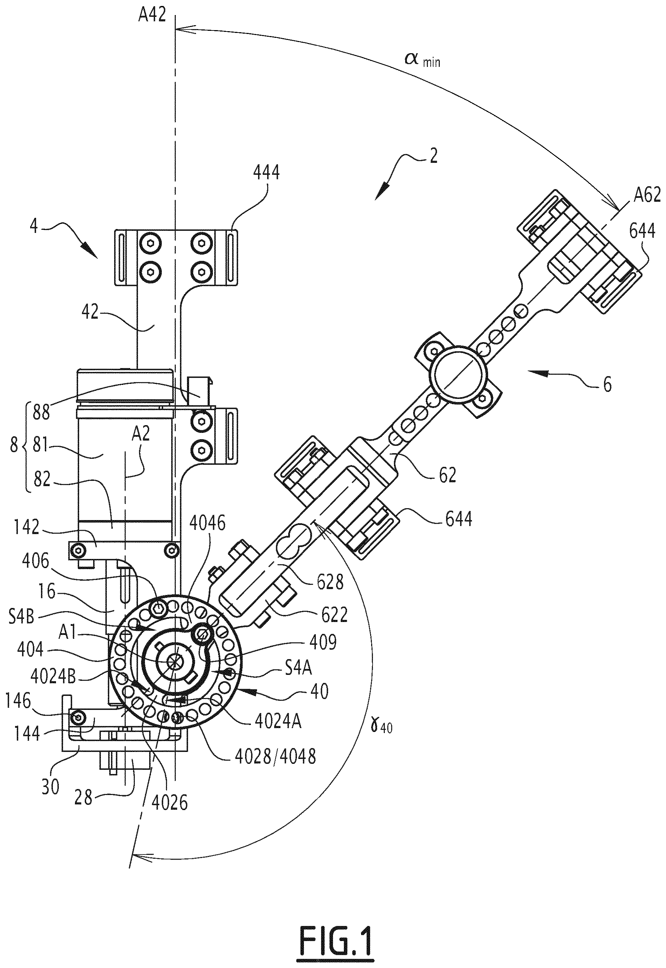

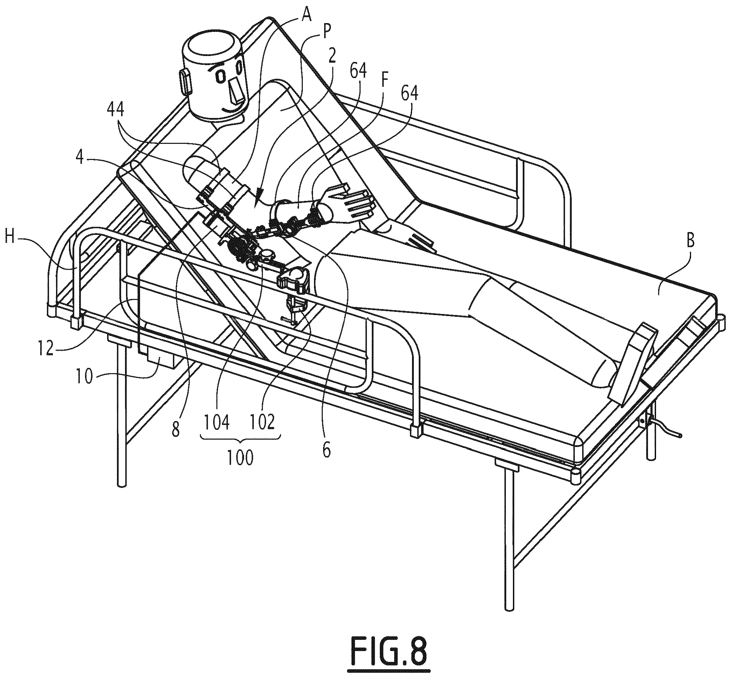

The elbow orthosis 2 represented on figures 1 to 8 includes a first fitting 4 configured for being mounted on an arm A of a patient P and a second fitting 6 configured for being mounted on the forearm F of the patient who, in the example of figure 8, is laying on a medical bed B.

Alternatively, the patient may be seated on a chair or stand.

The first fitting 4 includes an armature first 42, made of a metallic plate, and two braces 44. A42 denotes a longitudinal axis of the armature 42. Each brace 44 is immobilized on the armature 42 by four screws 46 cooperating with four nuts 48. Each screw 46 crosses two through holes 422 and 442 respectively provided in the armature 42 and in a base portion 444 of a brace 44.

The second fitting 6 includes another armature 62 and two braces 64. A62 denotes a longitudinal axis of the armature 62. The armature 62 includes a rigid elongated bracket 622 and two perforated rods 624 and 626 movable with respect to each other along the axis A62, in order to adapt the length of the second fitting 6 to the actual length of the patient's forearm. Each rod 624 or 626 is provided with a series of through holes 624A, respectively 626A. The rods 624 and 626 are guided with respect to each other by two clamps 625 held in position by two screws 627 cooperating with corresponding nuts 627A. A knob 629 is mounted on the clamps 625 and provided with a pin 629A adapted to penetrate in a group of two through holes 624A and 626A aligned along a direction perpendicular to the longitudinal axis A62, so as to fix the length of the sub-armature made of the rods 624 and 626.

A strain gauge 628 is inserted, along the longitudinal axis A62, between the bracket 622 and the sub-armature made of the rods 624 and 626. This strain gauge 628 measures the deflection of the armature 62 and translates it into torque at the level of an axis A1, which is a first rotation axis between the fittings 4 and 6. This allows detecting a torque applied, between the two fittings 4 and 6, on an articulation mechanism of the elbow orthosis 2.

A base portion 644 of each brace 64 is mounted at one end of a rod 624 or 626 by respective screws 66 cooperating with corresponding nuts 68. A pair of screws 66 is also used for fastening the strain gauge 628 to one of the rods, rod 626 in the example of the figures. Another pair of screws 66 is used for fastening the strain gauge 628 to the bracket 622.

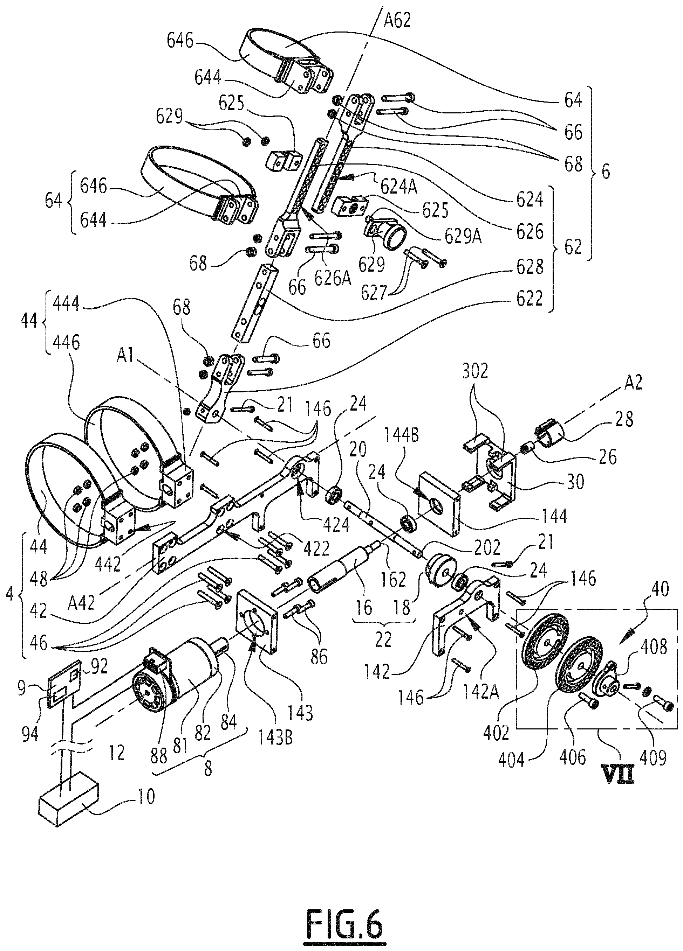

446 and 646 respectively denote a flexible strap of a brace 44 or 64. For the sake of simplicity, the straps 446 and 646 are represented on figures 6 and 8 only.

The two fittings 4 and 6 are articulated around the first rotation axis A1.

The two longitudinal axes A42 and A62 are respectively radial with respect to the first rotation axis A1.

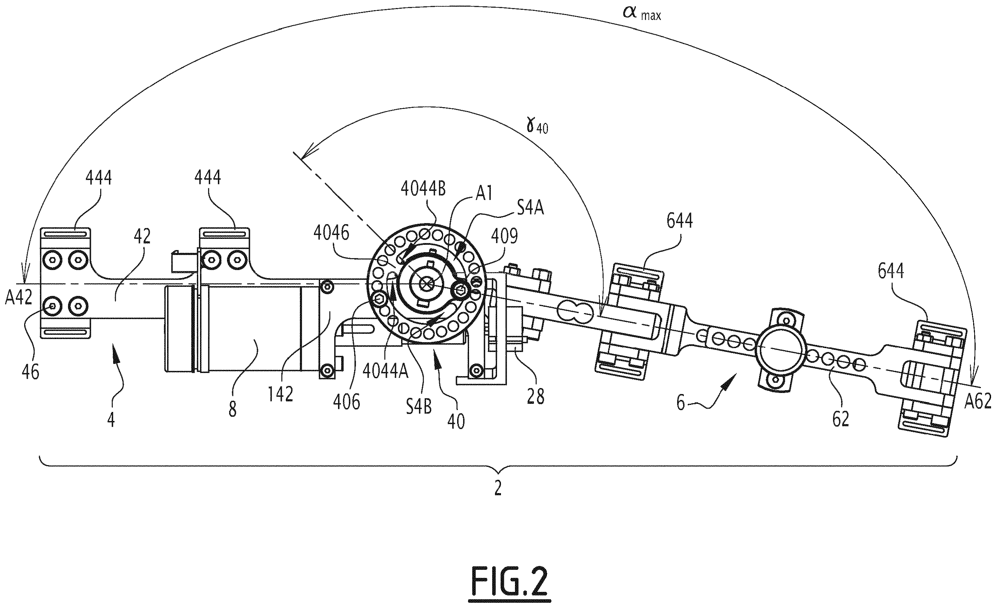

$\alpha$ denotes an angle measured between the two longitudinal axes A42 and A62 above the first rotation axis A1 in the right arm configuration represented on figures 1 and 2. This angle $\alpha$ can vary between a minimum value $\alpha_{min}$ corresponding to the flexed position, or folded position, of figure 1 and a maximum value $\alpha_{max}$ corresponding to the extended position of figure 2. It should be noted here that the maximum value $\alpha_{max}$ can be strictly greater than 180°, as shown in figure 2, which corresponds to some morphologies.

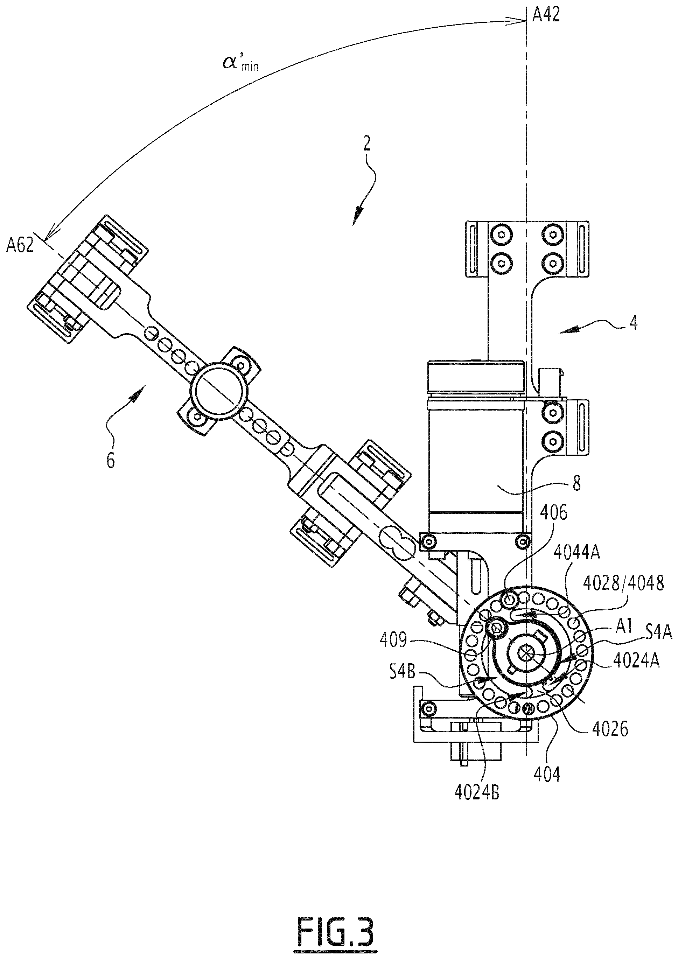

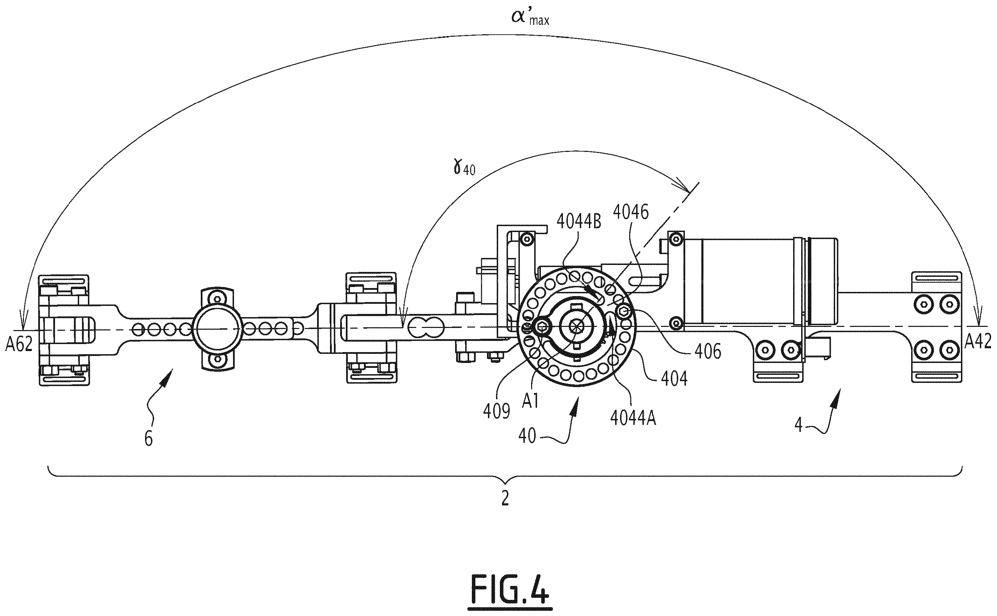

The minimum and maximum values for angle $\alpha$ when the orthosis 2 is configured for the left arm of a given patient can be equal to or different from the corresponding values in the configuration for a right arm, as shown respectively by values $\alpha'_{min}$ and $\alpha'_{max}$ on figures 3 and 4. Here value $\alpha'_{min}$ equals value $\alpha'_{min}$ and value $\alpha'_{max}$ is different from value $\alpha_{max}$.

An electric gear-motor 8 also belongs to the orthosis 2 and includes a brushless DC motor 81, a reducer 82 and an output shaft 84 aligned on a second rotation axis A2 perpendicular to, and non-secant with, the first rotation axis A1.

In the example of the figures, axes A2 and A42 are parallel. However, another relative orientation is possible.

Advantageously, the reducer 82 is an epicyclical reducer. However, another type of reducer can also be used.

The gear-motor 8 is capable of delivering a torque large enough to move the second fitting 6 with respect to the first fitting 4, in a flexion or extension movement of the elbow around the first rotation axis A1, even with no help of the patient, for instance when the spasticity of the patient's muscles prevents him or her to assist this movement. This is essential in order to help a patient to move his or her arm, even if he cannot do it with his or her own muscles, for instance in the few days after a cardio-vascular accident.

An electronic control unit or ECU 9, in the form of a printed circuit board, is coupled to the motor 81 and includes a microcontroller 92, a memory 94 and non-represented other electronic components, as needed. The electronic control unit is configured for piloting the electric gear-motor 8 and for supplying it with electric power coming from a battery set 10 via an electric line 12. For the sake of simplicity, items 9, 10 and 12 are represented on figures 6 and 8 only. The ECU 9 and the electric line 12 are connected to a connector 88 of the gear-motor 8.

Alternatively, in a non-represented alternative embodiment of the invention, instead of a battery set, one can use a mains supply for supplying the gear-motor with electric power.

A box-like structure 14 is made by four flat armatures cut in metallic flat panels, for instance made of aluminum or iron. In the example of the figures, the four armatures of the box-like structure 14 have the same thickness and are cut in the same aluminum panel. Alternatively, they can have different thicknesses.

The first armature of the box-like structure is formed by the armature 42. A second armature 142 of the box-like structure is formed by a C-shaped plate, which extends parallel to the first armature 42 in the mounted configuration of box-like structure 14. A third armature 143 and a fourth armature 144 of the box-like structure are made with square plates and extend between the first and second armatures 42, 142, perpendicular to these first and second armatures and to the second rotation axis A2. The third and fourth armatures 143 and 144 are parallel. The box-like structure has a globally parallelepiped shape. Each third or fourth armature 143 or 144 has a central circular through hole 143B, respectively 144B, aligned on the second rotation axis A2 in the mounted configuration of box-like structure 14 and of the orthosis 2.

The armatures 42, 142, 143 and 144 of the box-like structure 14 are held together by screws 146. On the other hand, the electric gear-motor 8 is mounted on the third armature 144 and immobilized thereon by four screws 86.

A threaded rod 16 is secured in rotation to the free end of the output shaft 84 and extends within the box-like structure 14, along the second rotation axis A2. This threaded rod 16 cooperates with a toothed wheel 18 mounted on, and secured in rotation with, a rotation shaft 20, which extends along the first rotation axis A1.

The connection in rotation around the first rotation axis A1, between the toothed wheel 18 and the rotation shaft 20, is made via a screw 21 inserted in two aligned housings respectively provided in the toothed wheel and in the rotation shaft. Alternatively, a key or a retractable pin can be used instead of the connection screw 21. In such a case, the key or pin is preferably force-fitted in the wheel 18 and/or in the shaft 20.

Parts 16 and 18 together form a worm-and-wheel transmission 22.

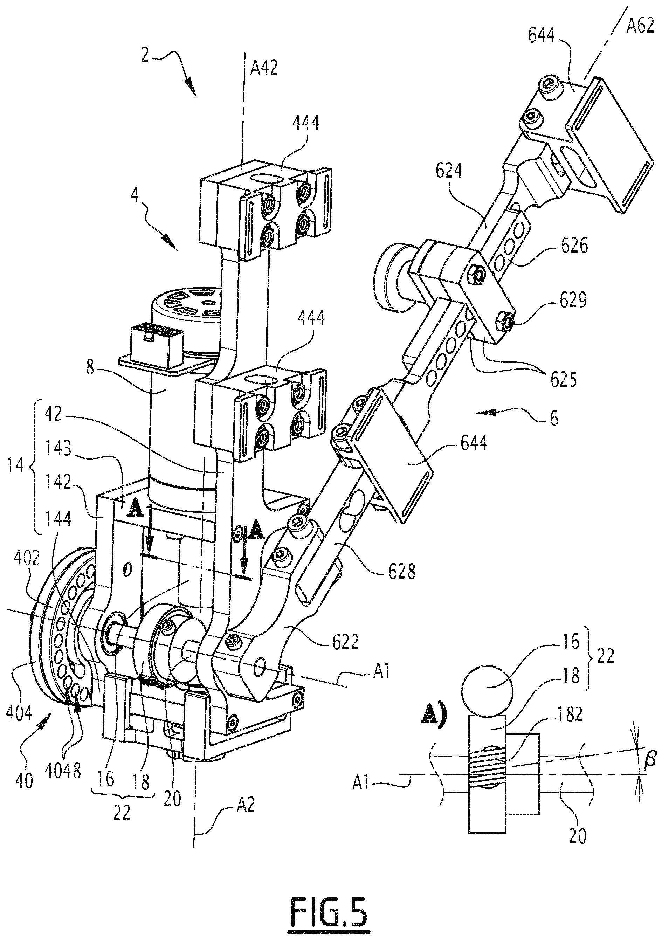

$\beta$ denotes an inclination angle between the teeth 182 of the toothed wheel 18 and the first rotation axis A1. This angle is visible on insert A) of figure 5 and chosen so that the worm-and-wheel transmission 22 is reversible. Due to the reversibility of the worm-and-wheel transmission, the threaded rod 16 can drive the toothed wheel 18 in a normal operation mode of the orthosis 2 and the toothed wheel can drive the threaded rod in a setting mode of the elbow orthosis, as explained here below. In practice, the angle $\beta$ is chosen above 10°

The rotation shaft 20 is supported within the box-like structure 14, namely between the first and second armatures 42 and 142, by two bearings 24 respectively mounted on the first and second plates.

The rotation shaft 20 crosses the first and second armatures 42 and 142. More precisely, the rotation shaft 20 crosses a through hole 424 of the first armature 42 and is secured to the extremity of the bracket 622 opposed to the strain gauge 628 via another screw 21, in such a way that the rotation shaft 20 and the other armature 62 rotate together around the first rotation axis A1. In other words, the bracket 622 of the second fitting 6 is secured to the rotation shaft 20 via the other screw 21. Alternatively, a key or a retractable pin can be used instead of the other connection screw 21. In such a case, the key or pin is preferably force-fitted in the wheel 18 and/or in the shaft 20.

A third bearing 24 supports the threaded rod 16 within the bore 144B. A cylindrical magnet 26 is mounted at the free end 162 of the threaded rod opposite to the output shaft 84 and secured on the threaded rod 16 in rotation around the second rotation axis A2.

Advantageously, the magnet 26 is glued onto the free end 162.

With the articulation mechanism made of sub-assemblies 14 and 22 and of the rotation shaft 20, it is possible to drive the second fitting 6 in rotation around the first rotation axis A1 by actuating the electric motor 8, so as to rotate the threaded rod 16, in one direction or the other, around the second rotation axis A2, thus the second fitting 6 around the first rotation axis.

An absolute rotation sensor 28 is mounted on a support member 30 attached to the fourth armature 144. With this respect, the support member 30 is provided with four flexible legs 302 hooked onto the fourth armature 144.

The rotation sensor 28 is mounted on the support member 30.

The absolute rotation sensor 28 allows detecting a rotation of the toothed rod 16 around the second rotation axis A2, thus to detect a rotation of the other armature 62 around the first rotation axis A1 with respect to the first armature 42, assuming that the worm-and-wheel transmission has a negligible play.

On the other hand, the strain gauge 628 allows detecting a torque applied on the articulation mechanism made of parts 14 and 22.

The output signal of the rotation sensor 28 is transferred to the electronic control unit 9 via a non-represented data line. Similarly, the output signal of the strain gauge 628 is transferred to the electronic control unit 9 via another non-represented data line.

The limits of the angular displacement of the second fitting 6 with respect to the first fitting 8 can be set electronically in the memory of the electronic control unit 9, via proper programming of this electronic control unit.

For the sake of safety, a mechanical stop arrangement 40 is used for limiting the angular movement of the second fitting 6 with respect to the first fitting 4, in particular in case of improper programming of the electronic control unit 9 or in case of a defective rotation sensor 28.

This mechanical stop arrangement 40 is supported by the box-like structure 14 and includes a first circular plate 402 and a second circular plate 404.

In the example of the figures, the two plates 402 and 404 are identical, which is advantageous in terms of manufacturing and maintenance. However, this is not compulsory.

The first plate 402 includes a first central bore 4022 centered on a central axis A402 of the first plate 402 and adapted to accommodate a portion of the rotation shaft 20. The first plate 402 includes first circular slot 4024 centered on the central axis A402. Similarly, the second plate 404 includes a central bore 4042, centered on a central axis A404 of the second plate 404 and adapted to accommodate a portion of the rotation shaft 20, and a circular slot 4044 in the form of an arc of a circle centered on the axis A404.

In mounted configuration of the orthosis 2, axes A1, A402 and A404 are superimposed and define a common central axis for the rotation shaft 20 and for the first and second plates 402 and 404. The two plates 402 and 404 are mounted next to each other along this common axis and the rotation shaft 20 crosses the central bores 4022 and 4042.

Each circular slot 4024 or 4044 extends around the corresponding central axis A402 or A404 on an angular sector whose apex angle $\delta_2$, respectively $\delta_4$, is strictly less than 360°, in practice less than 330°, so that a bridge 4026 or 4046 connects a central portion 4021 or 4041 of the plate 402 or 404, where the central bore 4022 or 4042 is located, to a peripheral portion 4023 or 4043 of the same plate.

Thus, the adjective "circular" used for qualifying the slots 4024 and 4044 does not means that they are annular, but that they extend each on an arc of a circle.

Here, angles $\delta_2$ and $\delta_4$ are identical since the first and second plates 402 and 404 are identical. However, this is not compulsory.

4024A and 4024B respectively denote the two extremities of the circular slot 4024 located on either side of the bridge 4026. Similarly, 4044A and 4044B respectively denote the two extremities of the circular slot 4044 located on either side of the bridge 4046.

When the first and second plates 402 and 404 are mounted on the rotation shaft 20, depending on their respective orientation around the axis A2, that is depending on the position of the bridges 4026 and 4046, a greater or lesser portion of the circular slots 4024 and 4044 overlap.

A circular series of twenty-four through holes 4028 or 4048 is drilled in each peripheral portion 4023 or 4043. Each series of twenty-four through holes 4028 or 4048 is centered of the central axis A402 or A404 of the corresponding plate 402 or 404.

The relative orientation of the first and second plates 402 and 404 around the first rotation axis A1, thus around the common axis, is set by rotating each plate 402 and 404 around its central axis A402, A404 aligned on the first rotation axis A1. Once the relative orientation of the first and second plates 402 and 404 is set, that is once the bridges 4026 and 4046 are correctly positioned, this orientation can be fixed by inserting a screw 406 into two aligned through holes 4028 and 4048 and by screwing this screw in a threaded hole 142A of the second armature 142. This setting of the relative orientation of the first and second plates 402 and 404 belongs to the action of configuring the elbow orthosis 2.

S4A denotes a first combined slot made through the first and second plates 402 and 404 by two overlapping portions of the circular slots 4024 and 4044. S4B denotes a second combined slot made through the first and second plates 402 and 404 by two other overlapping portions of the circular slots 4024 and 4044, on the opposite side of the bridges 4026 and 4046.

On the other hand, a crank 408 is secured at the end 202 of the rotation shaft 20 opposite to the bracket 622, via an additional screw 482. Alternatively, a key or a retractable pin can be used instead of the connection screw 482. In such a case, the key or pin is preferably force-fitted in the wheel 18 and/or in the shaft 20.

A pin 409 is mounted on the crank 408 with interposition of a washer 410 and crosses a through hole 4084 provided on the crank. When the crank 408 is mounted on the rotation shaft 20, the pin 409 extends parallel to the first rotation axis A1. This pin 409 penetrates into one of the combined slots S4A or S4B. Let's assume the pin 409 penetrates into the combined slot S4A, as shown on figures 1 and 2.

The angular movement of the pin 409 is limited by the stops formed by the extremities 4024A, 4024B, 4044A or 4044B of the overlapping portions of the circular slots 4024 and 4044. In practice, these extremities are defined by the side edges of the bridges 4026 and 4046.

As can be deduced by the comparison of figures 1 and 2, once the screw 406 has been screwed within the threaded hole 142A through the two aligned through holes 4028 and 4048 and once the pin has been introduced into the combined slot S4A, it can slide within the combined slot S4A when following the rotational movement of the rotation shaft, to which it is secured in rotation via the crank 408 and the screw 482.

The combined slot S4A extends on an arc of the circle having an apex angle $\gamma_{40}$ defined between the bridges 4026 and 4046, more precisely between the extremities 4024A and 4044A. This angle $\gamma_{40}$ is the amplitude of the maximum angular movement of the second fitting 6 with respect to the first fitting 4, around the first rotation axis A1.

This angle $\gamma_{40}$ is equal to the difference between the maximum and minimum angles $\alpha_{max}$ and $\alpha_{min}$ mentioned here-above.

Therefore, the following equation prevails:

$$\gamma_{40} = \alpha_{max} - \alpha_{min} \qquad \text{(equation 1)}$$

A proper definition of the orientation of the two plates 402 and 404 around the first rotation axis A1 allows setting the value of the angle $\gamma_{40}$, thus of the maximum amplitude of the relative movement between the first and second fittings 4 and 6. The simple structure of the mechanical stop arrangement makes it easy and intuitive to use for setting this angle $\gamma_{40}$.

The mechanical stop arrangement 40 is a safety guard and its configuration, that is the action of setting the angle $\gamma_{40}$, can be made in conjunction with the programming of the ECU 9, where control parameters can be set for limiting the movements of the output shaft 84. Thus, the mechanical stop arrangement 40 is used mainly in case of a default in the programming of the ECU 9 or a defective component of this ECU.

In addition, the simple and intuitive structure of the mechanical stop arrangement 40 allows quickly switching between the right arm configuration represented on figures 1 and 2 and the left arm configuration represented on figures 3 and 4, by changing the relative position of the first and second plates 402 and 404 around the first rotation axis A1 and the control parameters of the gear-motor 8 stored in the memory of the ECU 9.

In particular, it is possible to switch between a configuration of the elbow orthosis for a right arm and a configuration of the elbow orthosis for a left arm by inverting its most extended position and its most flexed position defined by the combined slots S4A and/or S4B, that is by the overlapping portions of the first and second circular slots 4024 and 4044.

According to an advantageous aspect of the invention, the reversibility of the worm-and-wheel transmission 22 can be used upon configuration of the elbow orthosis 2.

According to a first approach, once the mechanical stop arrangement 40 has been set the elbow orthosis 2 can be configured by moving the second fitting 6 with respect to the first fitting 4, in order to align the first fitting on the arm A of the patient P and the second fitting 6 on the forearm F of the patient, when the gear-motor 8 is not energized. This allows adjusting the opening angle of the elbow orthosis to the actual rest position of the patient's arm and forearm. Thereafter, the first and second fitting can be respectively secured to the patient's arm and forearm.

According to a second approach, still using the reversibility of the worm-and-wheel transmission 22 and once the mechanical stop arrangement 40 has been set and the braces 44 and 64 have been closed around the arm A and forearm F of the patient P, it is possible for a medical practitioner to move the forearm of a patient around the first articulation axis, when the gear-motor 8 is not energized, so as to bring the pin 409 into the extremities of the combined slot S4A, in contact with the bridges 4026 and 4046, while taking care of not hurting the patient. This allows checking that the two limit positions, defined by the ends of the combined slot S4A where the pin 409 is engaged, are compatible with the morphology and pathology of the patient and that the elbow orthosis 2 will not hurt the patient when the gear-motor is actuated.

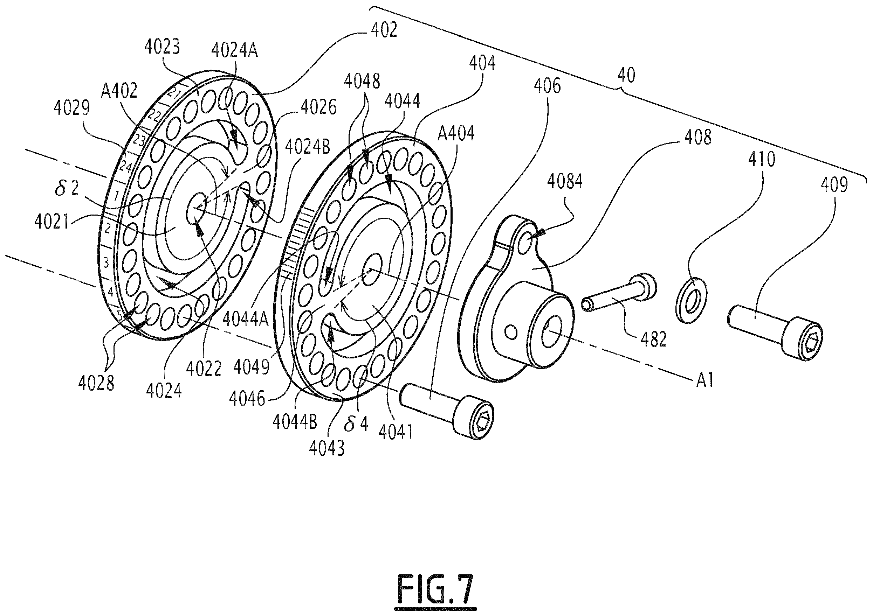

According to an optional aspect of the invention represented on figure 7 only, a graduation mark 4029 is provided on the outer edge of the first plate 402, here in the form of twenty-four numbers identifying each through hole 4028 located nearby. This allows identifying the angular position of the first plate with respect to the screw 406 or a non-represented mark provided on the second armature 142.

In a non-represented alternative embodiment of the invention, the graduation mark 4029 includes angular indications, instead of twenty-four numbers.

According to a non-represented variant, a graduation mark can also be provided on the outer edge of the second plate 404, also here in the form of twenty-four numbers. Then the relative orientation of the two plates 402 and 404 around the first rotation axis A1 can be identified by a combination of two numbers or of angular indications visible on their edges, near the screw 406.

According to another optional aspect of the invention represented on figure 7 only, a knurled zone 4049 is provided on the outer edge of second plate 404. This knurled zone facilitates the application of a torque on the second plate, around its central axis A404, during setting of the configuration of the mechanical stop arrangement 40.

As shown on figure 7, the knurled zone 4049 extends on a limited angular sector of the outer edge of the second plate 404. In a variant, the knurled zone 4049 extends overall this edge, on 360°.

According to a non-represented variant, a knurled zone can also be provided on the outer edge of the first plate 402.

The graduation mark 4029 and the knurled zone 4409 can also be combined.

In a non-represented alternative embodiment of the invention, the graduation mark 4029 and/or he knurled zone 4049 is located outside the outer edge of the peripheral portion 4023 and/or 4043.

As represented on figure 8, a support member 100 is provided of supporting the elbow orthosis 2 from a handrail H of the medical bed B. This avoids the patient having to bear the weight of the orthosis. This support member 100 includes an anchoring part 102 configured for being locked on the handrail H and a bracket 104 articulated on the anchoring part 102 and provided with a clamp for holding a part of the orthosis, for instance the box-like structure 14. This allows the support member 100 to support at least items 4, 6, 8, 14, 20 and 22.

The support member 100 is adapted to the environment of the patient P. For instance, if the patient is seated in a wheelchair, the anchoring part 102 is configured for being fixed on an armrest of the wheelchair.

The battery set 10 is also mounted on the medical bed B and connected to the gear-motor via the connection line 12 formed of a bundle of electric wires, which relieves the patient of supporting the weight of this battery set. However, alternatively, the battery set 10 can be supported by one of the first and second fittings 4 and 6.

If a mains supply is used, as considered here above, no battery set needs to be supported and the electrical line might be plugged on a wall socket or on a current converter.

The embodiment, variants and optional aspects listed here-above may be combined to generate other embodiments of the invention defined in the appended claims.

**Claims**

1. An elbow orthosis (2) comprising a first fitting (4) configured to be mounted on an arm (A) of a patient (P), a second fitting (6) configured for being mounted on a forearm (F) of the patient, an articulation mechanism (14, 20, 22) between the first and second fittings and an electric gear-motor (8) mounted on the first fitting for moving the second fitting with respect to the first fitting in rotation around a first rotation axis (A1) of the articulation mechanism, wherein

   - an output shaft (84) of the electric gear-motor is aligned on a second rotation axis (A2) perpendicular to the first rotation axis and
   - the orthosis is equipped with

      • an angular sensor (28) for sensing the angular position of the second fitting (6) with respect to the first fitting (4) around the first rotation axis (A1);
      • a torque sensor (628) for sensing a torque exerted on the articulation (14, 22) mechanism by the electric gear-motor (8); and
      • a rotation shaft (20);

   - a worm-and-wheel transmission (22) drives the rotation shaft (20), which defines the first rotation axis, from the rotational movement of the output shaft (84) of the electric gear-motor;
   - the second fitting (6) is secured to the rotation shaft; and
   - a mechanical stop arrangement (40) is configured for limiting the angular movement of the second fitting (6) with respect to the first fitting (4) around the first rotation axis (A1),
   - the mechanical stop arrangement (40) includes

      o a first plate (402), with a first circular slot (4024), and
      o a crank (408) secured to the rotation shaft (20) and equipped with a pin (409) engaged in the first circular slot (4024);

**characterized in that**

- the first plate (402) is provided with a first series of through holes (4028) spread on a circle centered on a central axis (A402) of the first circular slot;

- the mechanical stop arrangement (40) includes a second plate (404) provided with a second circular slot (4044) and a second series of through holes (4048) spread on a circle centered on a central axis (A404) of the second circular slot; and

- the pin (409) of the crank (408) is engaged in the second circular slot (4024);

- the central axis (A402) of the first circular slot (4024) and the central axis (A404) of the second circular slot (4044) of the first and second plates (402, 404) are aligned on the first rotation axis (A1) and the relative orientation of the first and second plates (402, 404) around this first rotation axis is set by aligning at least one through hole (4028) of the first series with one through hole (4048) of the second series;

- some portions of the first and second circular slots (4024, 4044) overlap, depending on the orientation of the first and second plates around the common axis; and

- the respective extremities (4024A, 4024B, 4044A, 4044B) of the overlapping portions (S4A, S4B) of the first and second circular slots (4024, 4044) define the amplitude ($\gamma_{40}$) of the maximum angular movement of the second fitting (6) with respect to the first fitting (4), around the first rotation axis (A1), when it is driven by the worm-and-wheel transmission (22).

2. The elbow orthosis of claim 1, wherein the worm-and-wheel transmission (22) is reversible, in such a way that a threaded rod (16) of the worm-and-wheel transmission can drive a toothed wheel (18) of the worm-and-wheel transmission in a normal operation mode of the orthosis (2) and the toothed wheel can drive the threaded rod in a setting mode of the orthosis.

3. The elbow orthosis of claim 2, wherein an inclination angle ($\beta$) between the teeth (182) of the toothed wheel (18) and the first rotation axis (A1) is above 10°.

4. The elbow orthosis of any preceding claim, wherein it is configurable for use on a right or left arm, by changing the relative angular position of the first plate (402) with respect to the second plate (404) and some control parameters of the electric gear-motor (8).

5. The elbow orthosis of any preceding claim, wherein it includes a box-like structure (14) for supporting the gear-motor (8), the worm-and-wheel transmission (22), the rotation shaft (20) and the mechanical stop arrangement (40).

6. The elbow orthosis of claim 5, wherein the box-like structure (14) includes four armatures (42, 142, 143, 144) assembled together by screws (146), wherein the worm-and-wheel transmission (22) is mounted within the box-like structure, wherein the wheel (18) of the worm-and-wheel transmission is mounted on the rotation shaft (20) between two parallel armatures (42, 142) of the box-like structure and wherein the rotation shaft (20) is mounted on these two parallel armatures with a possibility of rotation.

7. The elbow orthosis of any preceding claim, wherein at least one of the first and second plates (402, 404) includes a graduation mark (4029) for identifying the relative orientation of the first and second plates.

8. The elbow orthosis of claim 7, wherein the graduation mark (4029) is formed on an edge of the at least one plate (402, 404).

9. The elbow orthosis of any preceding claim, wherein the first and/or second plate (402, 404) is provided with a knurled zone (4049).

10. The elbow orthosis of any preceding claim, wherein it includes a support member (100) for supporting at least the first and second fittings (4, 6), the electric gear-motor (8), the articulation mechanism (14, 22), the angular sensor (28), the torque sensor (628) and the worm-and-wheel transmission (22) with respect to a structure (H).

11. The elbow orthosis of any preceding claim, wherein it includes a battery set (10) for providing electric power to the electric gear-motor (8) and to an electronic control unit (9) for controlling the electric gear-motor and wherein the battery set is either supported by one of the first and second fittings (4, 6) or located in the vicinity of the first and second fittings and connected to the electric gear-motor via a bundle of electrical wires (12).

12. A process for adapting a configuration of an elbow orthosis (2) according to any preceding claim to the morphology and/or pathology of a patient (P), this method including at least the following steps:

a) adjusting the respective angular position of the first and second plates (402, 404) around their common central axis (A1, A402, A404), so as to define with their overlapping first and second circular slots a path (S4A, S4B) for the pin (409) mounted on the crank (408) secured to the rotation shaft (20); and
b) immobilizing the first and second plates in their respective angular positions by insertion of a locking member (406) in one through hole (4028) of the first series and in one through hole (4048) of the second series.

13. The process of claim 12, wherein the elbow orthosis is according to claim 2 and the process includes a further step consisting in:

c) moving the second fitting (6) with respect to the first fitting (4), in rotation around the first rotation axis (A1), in order to align the first fitting on the arm (A) of the patient (P) and the second fitting on the forearm (F) of the patient when the electric gear-motor is not energized, using the reversibility of the worm-and-wheel transmission; and
d) securing the first fitting (4) to the arm of the patient and the second fitting (6) to the forearm of the patient.

14. The process of one of claims 12 and 13, wherein the elbow orthosis (2) is according to claim 4 and wherein the passage of a configuration for a left arm to a configuration for a right arm, or vice versa, occurs by inverting the most extended position and the most flexed position of the orthosis defined by the overlapping portions (S4A, S4B) of the first and second circulars slots (4024, 4044).

15. The process of one of claims 12 to 14, wherein it includes a supplementary step implemented after b) and consisting in:
e) moving the second fitting (6) with respect to the first fitting (4) around the first rotation axis (A1), while the electric gear-motor (8) is not energized, up to bringing the pin (409) into the respective extremities (4024A, 4024B, 4044A, 4044B) of the overlapping portion (S4A, S4B) of the first and second circular slots (4024, 4044), in order to assess the maximum relative angular movement of the first and second fitting around the first rotation axis.


**Patentansprüche**

1. Ellenbogenorthese (2), umfassend einen ersten Beschlag (4), der so ausgebildet ist, dass er an einem Arm (A) eines Patienten (P) montiert werden kann, einen zweiten Beschlag (6), der so ausgebildet ist, dass er an einem Unterarm (F) des Patienten montiert werden kann, einen Gelenkmechanismus (14, 20, 22) zwischen dem ersten und dem zweiten Beschlag und einen elektrischen Getriebemotor (8), der an dem ersten Beschlag montiert ist, um den zweiten Beschlag in Bezug auf den ersten Beschlag in Drehung um eine erste Drehachse (A1) des Gelenkmechanismus zu bewegen, wobei

- eine Abtriebswelle (84) des elektrischen Getriebemotors auf einer zweiten Drehachse (A2) senkrecht zu der ersten Drehachse ausgerichtet ist und
- die Orthese mit

• einem Winkelsensor (28) zum Erfassen der Winkelposition des zweiten Beschlags (6) in Bezug auf den ersten Beschlag (4) um die erste Drehachse (A1);
• einem Drehmomentsensor (628) zum Erfassen eines Drehmoments, das von dem elektrischen Getriebemotor (8) auf den Gelenkmechanismus (14, 22) ausgeübt wird; und
• einer Drehwelle (20) ausgestattet ist;

- ein Schneckenradgetriebe (22) die Drehwelle (20), die die erste Drehachse definiert, aus der Drehbewegung der Abtriebswelle (84) des elektrischen Getriebemotors antreibt;
- der zweite Beschlag (6) an der Drehwelle befestigt ist; und
- eine mechanische Anschlaganordnung (40) zum Begrenzen der Winkelbewegung des zweiten Beschlags (6) in Bezug auf den ersten Beschlag (4) um die erste Drehachse (A1) ausgebildet ist,
- die mechanische Anschlaganordnung (40)

o eine erste Platte (402), mit einem ersten kreisförmigen Schlitz (4024), und
o eine Kurbel (408) einschließt, die an der Drehwelle (20) befestigt und mit einem Stift (409) ausgestattet ist, der in den ersten kreisförmigen Schlitz (4024) eingreift;
**dadurch gekennzeichnet, dass**

- die erste Platte (402) mit einer ersten Reihe von Durchgangslöchern (4028) versehen ist, die auf einem Kreis verteilt sind, der auf einer Zentralachse (A402) des ersten kreisförmigen Schlitzes zentriert ist;

- die mechanische Anschlaganordnung (40) eine zweite Platte (404) mit einem zweiten kreisförmigen Schlitz (4044) und einer zweiten Reihe von Durchgangslöchern (4048) einschließt, die auf einem Kreis verteilt sind, der auf einer Zentralachse (A404) des zweiten kreisförmigen Schlitzes zentriert ist; und

- der Stift (409) der Kurbel (408) in den zweiten kreisförmigen Schlitz (4024) eingreift;

- die Zentralachse (A402) des ersten kreisförmigen Schlitzes (4024) und die Zentralachse (A404) des zweiten kreisförmigen Schlitzes (4044) der ersten und zweiten Platte (402, 404) auf der ersten Drehachse (A1) ausgerichtet sind und die relative Ausrichtung der ersten und zweiten Platte (402, 404) um diese erste Drehachse eingestellt wird, indem mindestens ein Durchgangsloch (4028) der ersten Reihe mit einem Durchgangsloch (4048) der zweiten Reihe ausgerichtet wird;

- sich einige Abschnitte des ersten und zweiten kreisförmigen Schlitzes (4024, 4044) überlappen, abhängig von der Ausrichtung der ersten und zweiten Platte um die gemeinsame Achse; und

- die jeweiligen Enden (4024A, 4024B, 4044A, 4044B) der überlappenden Abschnitte (S4A, S4B) des ersten und zweiten kreisförmigen Schlitzes (4024, 4044) die Amplitude ($\gamma_{40}$) der maximalen Winkelbewegung des zweiten Beschlags (6) im Verhältnis zu dem ersten Beschlag (4) um die erste Drehachse (A1) definieren, wenn sie durch das Schneckenradgetriebe (22) angetrieben wird.

2. Ellenbogenorthese nach Anspruch 1, wobei das Schneckenradgetriebe (22) umkehrbar ist, so dass eine Gewindestange (16) des Schneckenradgetriebes ein Zahnrad (18) des Schneckenradgetriebes in einem Normalbetriebsmodus der Orthese (2) antreiben kann und das Zahnrad die Gewindestange in einem Einstellmodus der Orthese antreiben kann.

3. Ellenbogenorthese nach Anspruch 2, wobei ein Neigungswinkel ($\beta$) zwischen den Zähnen (182) des Zahnrades (18) und der ersten Drehachse (A1) größer als 10° ist.

4. Ellenbogenorthese nach einem der vorhergehenden Ansprüche, wobei sie für die Verwendung an einem rechten oder linken Arm konfigurierbar ist, indem sie die relative Winkelposition der ersten Platte (402) in Bezug auf die zweite Platte (404) und einige Steuerparameter des elektrischen Getriebemotors (8) ändert.

5. Ellenbogenorthese nach einem der vorhergehenden Ansprüche, wobei sie eine kastenartige Struktur (14) zum Abstützen des Getriebemotors (8), des Schneckenradgetriebes (22), der Drehwelle (20) und der mechanischen Anschlaganordnung (40) einschließt.

6. Ellenbogenorthese nach Anspruch 5, wobei die kastenartige Struktur (14) vier Anker (42, 142, 143, 144) einschließt, die mit Schrauben (146) zusammengebaut sind, wobei das Schneckenradgetriebe (22) innerhalb der kastenartigen Struktur montiert ist, wobei das Rad (18) des Schneckenradgetriebes auf der Drehwelle (20) zwischen zwei parallelen Ankern (42, 142) der kastenartigen Struktur montiert ist und wobei die Drehwelle (20) auf diesen zwei parallelen Ankern mit einer Drehmöglichkeit montiert ist.

7. Ellenbogenorthese nach einem der vorhergehenden Ansprüche, wobei mindestens eine der ersten und zweiten Platte (402, 404) eine Abstufungsmarkierung (4029) zum Identifizieren der relativen Ausrichtung der ersten und zweiten Platte aufweist.

8. Ellenbogenorthese nach Anspruch 7, wobei die Abstufungsmarkierung (4029) an einer Kante der mindestens einen Platte (402, 404) ausgebildet ist.

9. Ellenbogenorthese nach einem der vorhergehenden Ansprüche, wobei die erste und/oder zweite Platte (402, 404) mit einem gerändelten Bereich (4049) versehen ist.

10. Ellenbogenorthese nach einem der vorhergehenden Ansprüche, wobei sie ein Stützelement (100) zum Abstützen mindestens des ersten und zweiten Beschlags (4, 6), des elektrischen Getriebemotors (8), des Gelenkmechanismus (14, 22), des Winkelsensors (28), des Drehmomentsensors (628) und des Schneckenradgetriebes (22) in Bezug auf eine Struktur (H) einschließt.

11. Ellenbogenorthese nach einem der vorhergehenden Ansprüche, wobei sie einen Batteriesatz (10) zum Bereitstellen von elektrischer Energie für den elektrischen Getriebemotor (8) und für eine elektronische Steuereinheit (9) zum Steuern des elektrischen Getriebemotors einschließt und wobei der Batteriesatz entweder von einem des ersten und zweiten Beschlags (4, 6) abgestützt wird oder sich in der Nähe des ersten und zweiten Beschlags befindet und über ein Bündel elektrischer Leitungen (12) mit dem elektrischen Getriebemotor verbunden ist.

12. Verfahren zum Anpassen einer Konfiguration einer Ellenbogenorthese (2) nach einem der vorhergehenden Ansprüche an die Morphologie und/oder Pathologie eines Patienten (P), wobei dieses Verfahren mindestens die folgenden Schritte einschließt:

a) Einstellen der jeweiligen Winkelposition der ersten und zweiten Platte (402, 404) um ihre gemeinsame Zentralachse (A1, A402, A404), um mit ihrem überlappenden ersten und zweiten kreisförmigen Schlitz einen Pfad (S4A, S4B) für den an der Kurbel (408) befestigten und an der Drehwelle (20) gesicherten Stift (409) zu definieren; und
b) Immobilisieren der ersten und zweiten Platte in ihren jeweiligen Winkelpositionen durch Einsetzen eines Verriegelungselements (406) in ein Durchgangsloch (4028) der ersten Reihe und in ein Durchgangsloch (4048) der zweiten Reihe.

13. Verfahren nach Anspruch 12, wobei die Ellenbogenorthese nach Anspruch 2 ausgeführt ist und das Verfahren einen weiteren Schritt einschließt, der aus Folgendem besteht:

c) Bewegen des zweiten Beschlags (6) in Bezug auf den ersten Beschlag (4) in Drehung um die erste Drehachse (A1), um den ersten Beschlag an dem Arm (A) des Patienten (P) und den zweiten Beschlag an dem Unterarm (F) des Patienten auszurichten, wenn der Elektrogetriebemotor nicht bestromt ist, unter Verwendung der Umkehrbarkeit des Schneckenradgetriebes; und
d) Sichern des ersten Beschlags (4) an dem Arm des Patienten und des zweiten Beschlags (6) an dem Unterarm des Patienten.

14. Verfahren nach einem der Ansprüche 12 und 13, wobei die Ellenbogenorthese (2) nach Anspruch 4 ausgeführt ist und wobei der Übergang einer Konfiguration für einen linken Arm zu einer Konfiguration für einen rechten Arm oder umgekehrt erfolgt, indem die am weitesten ausgefahrene Position und die am weitesten gebogene Position der Orthese, die durch die überlappenden Abschnitte (S4A, S4B) des ersten und zweiten kreisförmigen Schlitzes (4024, 4044) definiert sind, umgekehrt werden.

15. Verfahren nach einem der Ansprüche 12 bis 14, wobei es einen nach b) implementierten Zusatzschritt einschließt und aus Folgendem besteht:
e) Bewegen des zweiten Beschlags (6) in Bezug auf den ersten Beschlag (4) um die erste Drehachse (A1), während der Elektrogetriebemotor (8) nicht bestromt ist, bis der Stift (409) in die jeweiligen Enden (4024A, 4024B, 4044A, 4044B) des überlappenden Abschnitts (S4A, S4B) des ersten und zweiten kreisförmigen Schlitzes (4024, 4044) gebracht wird, um die maximale relative Winkelbewegung des ersten und zweiten Beschlags um die erste Drehachse zu beurteilen.

**Revendications**

1. Orthèse (2) de coude comprenant un premier raccord (4) configuré pour être monté sur un bras (A) d'un patient (P), un second raccord (6) configuré pour être monté sur un avant-bras (F) du patient, un mécanisme d'articulation (14, 20, 22) entre les premier et second raccords et un motoréducteur électrique (8) monté sur le premier raccord pour déplacer le second raccord par rapport au premier raccord en rotation autour d'un premier axe de rotation (A1) du mécanisme d'articulation, dans laquelle

- un arbre de sortie (84) du motoréducteur électrique est aligné sur un second axe de rotation (A2) perpendiculaire au premier axe de rotation et
- l'orthèse est équipée

• d'un capteur angulaire (28) pour détecter la position angulaire du second raccord (6) par rapport au premier raccord (4) autour du premier axe de rotation (A1) ;
• d'un capteur de couple (628) pour détecter un couple exercé sur le mécanisme d'articulation (14, 22) par le motoréducteur électrique (8) ; et
• d'un arbre de rotation (20) ;

- une transmission à vis sans fin (22) entraîne l'arbre de rotation (20), qui définit le premier axe de rotation, à partir du mouvement de rotation de l'arbre de sortie (84) du motoréducteur électrique ;
- le second raccord (6) est fixé à l'arbre de rotation ; et
- un agencement d'arrêt mécanique (40) est configuré pour limiter le mouvement angulaire du second raccord (6) par rapport au premier raccord (4) autour du premier axe de rotation (A1),
- l'agencement d'arrêt mécanique (40) comporte

  o une première plaque (402), avec une première fente circulaire (4024), et
  o une manivelle (408) fixée à l'arbre de rotation (20) et équipée d'une broche (409) mise en prise dans la première fente circulaire (4024) ;
  **caractérisée en ce que**

- la première plaque (402) est pourvue d'une première série de trous traversants (4028) répartis sur un cercle centré sur un axe central (A402) de la première fente circulaire ;
- l'agencement d'arrêt mécanique (40) comporte une seconde plaque (404) pourvue d'une seconde fente circulaire (4044) et d'une seconde série de trous traversants (4048) répartis sur un cercle centré sur un axe central (A404) de la seconde fente circulaire ; et
- la broche (409) de la manivelle (408) est mise en prise dans la seconde fente circulaire (4024) ;
- l'axe central (A402) de la première fente circulaire (4024) et l'axe central (A404) de la seconde fente circulaire (4044) des première et seconde plaques (402, 404) sont alignés sur le premier axe de rotation (A1) et l'orientation relative des première et seconde plaques (402, 404) autour de ce premier axe de rotation est réglée en alignant au moins un trou traversant (4028) de la première série avec un trou traversant (4048) de la seconde série ;
- certaines portions des première et seconde fentes circulaires (4024, 4044) se chevauchent, en fonction de l'orientation des première et seconde plaques autour de l'axe commun ; et
- les extrémités respectives (4024A, 4024B, 4044A, 4044B) des portions de chevauchement (S4A, S4B) des première et seconde fentes circulaires (4024, 4044) définissent l'amplitude ($\gamma_{40}$) du mouvement angulaire maximal du second raccord (6) par rapport au premier raccord (4), autour du premier axe de rotation (A1), lorsqu'il est entraîné par la transmission à vis sans fin (22).

2. Orthèse de coude selon la revendication 1, dans laquelle la transmission à vis sans fin (22) est réversible, de sorte qu'une tige filetée (16) de la transmission à vis sans fin peut entraîner une roue dentée (18) de la transmission à vis sans fin dans un mode de fonctionnement normal de l'orthèse (2) et la roue dentée peut entraîner la tige filetée dans un mode de réglage de l'orthèse.

3. Orthèse de coude selon la revendication 2, dans laquelle un angle d'inclinaison ($\beta$) entre les dents (182) de la roue dentée (18) et le premier axe de rotation (A1) est supérieur à 10°.

4. Orthèse de coude selon une quelconque revendication précédente, dans laquelle elle est configurable pour une utilisation sur un bras droit ou gauche, en modifiant la position angulaire relative de la première plaque (402) par rapport à la seconde plaque (404) et certains paramètres de commande du motoréducteur électrique (8).

5. Orthèse de coude selon une quelconque revendication précédente, dans laquelle elle comporte une structure (14) de type boîte pour supporter le motoréducteur (8), la transmission à vis sans fin (22), l'arbre de rotation (20) et l'agencement d'arrêt mécanique (40).

6. Orthèse de coude selon la revendication 5, dans laquelle la structure (14) de type de boîte comporte quatre armatures (42, 142, 143, 144) assemblées ensemble par des vis (146), dans laquelle la transmission à vis sans fin (22) est montée à l'intérieur de la structure de type boîte, dans laquelle la roue (18) de la transmission à vis sans fin est montée sur l'arbre de rotation (20) entre deux armatures (42, 142) parallèles de la structure de type boîte et dans laquelle l'arbre de rotation (20) est monté sur ces deux armatures parallèles avec une possibilité de rotation.

7. Orthèse de coude selon une quelconque revendication précédente, dans laquelle au moins l'une des première et seconde plaques (402, 404) comporte une marque de graduation (4029) pour identifier l'orientation relative des première et seconde plaques.

8. Orthèse de coude selon la revendication 7, dans laquelle la marque de graduation (4029) est formée sur un bord de l'au moins une plaque (402, 404).

**9.** Orthèse de coude selon une quelconque revendication précédente, dans laquelle la première et/ou la seconde plaque (402, 404) sont pourvues d'une zone moletée (4049).

**10.** Orthèse de coude selon une quelconque revendication précédente, dans laquelle elle comporte un organe de support (100) pour supporter au moins les premier et second raccords (4, 6), le motoréducteur électrique (8), le mécanisme d'articulation (14, 22), le capteur angulaire (28), le capteur de couple (628) et la transmission à vis sans fin (22) par rapport à une structure (H).

**11.** Orthèse de coude selon une quelconque revendication précédente, dans laquelle elle comporte un jeu de batteries (10) pour fournir une alimentation électrique au motoréducteur électrique (8) et à une unité de commande électronique (9) pour commander le motoréducteur électrique et dans laquelle le jeu de batteries est soit supporté par l'un parmi les premier et second raccords (4, 6), soit situé à proximité des premier et second raccords et connecté au motoréducteur électrique par l'intermédiaire d'un faisceau de fils électriques (12).

**12.** Procédé d'adaptation d'une configuration d'orthèse (2) de coude selon une quelconque revendication précédente à la morphologie et/ou à la pathologie d'un patient (P), ledit procédé comportant au moins les étapes suivantes :

a) l'ajustement de la position angulaire respective des première et seconde plaques (402, 404) autour de leur axe central (A1, A402, A404) commun, de manière à définir avec leurs première et seconde fentes circulaires se chevauchant une voie (S4A, S4B) pour la broche (409) montée sur la manivelle (408) fixée à l'arbre de rotation (20) ; et
b) l'immobilisation des première et seconde plaques dans leurs positions angulaires respectives par insertion d'un organe de verrouillage (406) dans un trou traversant (4028) de la première série et dans un trou traversant (4048) de la seconde série.

**13.** Procédé selon la revendication 12, dans lequel l'orthèse de coude est selon la revendication 2 et le procédé comporte une étape supplémentaire consistant à :

c) déplacer le second raccord (6) par rapport au premier raccord (4), en rotation autour du premier axe de rotation (A1), afin d'aligner le premier raccord sur le bras (A) du patient (P) et le second raccord sur l'avant-bras (F) du patient lorsque le motoréducteur électrique n'est pas alimenté, en utilisant la réversibilité de la transmission à vis sans fin ; et
d) fixer le premier raccord (4) au bras du patient et le second raccord (6) à l'avant-bras du patient.

**14.** Procédé selon l'une des revendications 12 et 13, dans lequel l'orthèse (2) de coude est selon la revendication 4 et dans lequel le passage d'une configuration pour un bras gauche à une configuration pour un bras droit, ou inversement, s'effectue en inversant la position la plus étendue et la position la plus fléchie de l'orthèse définies par les portions de chevauchement (S4A, S4B) des première et seconde fentes circulaires (4024, 4044).

**15.** Procédé selon l'une des revendications 12 à 14, dans lequel il comporte une étape supplémentaire mise en œuvre après l'étape b) et consistant à :
e) déplacer le second raccord (6) par rapport au premier raccord (4) autour du premier axe de rotation (A1), alors que le motoréducteur électrique (8) n'est pas alimenté, jusqu'à amener la broche (409) dans les extrémités respectives (4024A, 4024B, 4044A, 4044B) de la portion de chevauchement (S4A, S4B) des première et seconde fentes circulaires (4024, 4044), afin d'évaluer le mouvement angulaire relatif maximal du premier et du second raccord autour du premier axe de rotation.

## FIG.1

FIG.2

A42

α'min

A62

2

4

6

8

406

4044A

4028/4048

409

A1    S4A

S4B

4024A

4026

4024B

404

## FIG.3

EP 4 422 565 B1

$\alpha'_{max}$

$\gamma_{40}$

4044B

4046

406

404

409

A1

4044A

40

4

A62

A42

6

2

## FIG.4

## FIG.5

**FIG.6**

FIG.7

FIG.8

**EP 4 422 565 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6203511 B1
- US 6993808 B
- EP 314970 A
- KR 20200067446

**Non-patent literature cited in the description**

- **RIPLE et al.** *International Journal of Advanced Robotic Systems*, 2014, vol. 11, 143